(19) **Europäisches Patentamt**
**European Patent Office**
**Office européen des brevets**

(11) **EP 1 582 090 B1**

(12) **EUROPEAN PATENT SPECIFICATION**

(45) Date of publication and mention of the grant of the patent:
**12.10.2011 Bulletin 2011/41**

(51) Int Cl.:
**H04S 1/00** (2006.01)

(21) Application number: **03813955.6**

(22) Date of filing: **04.12.2003**

(86) International application number:
**PCT/IB2003/005925**

(87) International publication number:
**WO 2004/058059 (15.07.2004 Gazette 2004/29)**

(54) **AUDIO REPRODUCTION APPARATUS, FEEDBACK SYSTEM AND METHOD**

AUDIOWIEDERGABEVORRICHTUNG,RÜCKKOPPLUNGSSYSTEM UND VERFAHREN

APPAREIL DE REPRODUCTION AUDIO, SYSTEME ET PROCEDE DE RETROACTION

(84) Designated Contracting States:
**AT BE BG CH CY CZ DE DK EE ES FI FR GB GR HU IE IT LI LU MC NL PT RO SE SI SK TR**

(30) Priority: **30.12.2002 EP 02080569**

(43) Date of publication of application:
**05.10.2005 Bulletin 2005/40**

(73) Proprietor: **Koninklijke Philips Electronics N.V. 5621 BA Eindhoven (NL)**

(72) Inventors:
• **AARTS, Ronaldus, M.**
**NL-5656 AA Eindhoven (NL)**
• **GOUGH, Paul, A.**
**NL-5656 AA Eindhoven (NL)**
• **SCHOBBEN, Daniel, W., E.**
**NL-5656 AA Eindhoven (NL)**

(74) Representative: **Groenendaal, Antonius W. M. Philips**
**Intellectual Property & Standards**
**P.O. Box 220**
**5600 AE Eindhoven (NL)**

(56) References cited:
**US-A- 4 110 918      US-A- 4 788 983**
**US-B1- 6 195 434**

• **PATENT ABSTRACTS OF JAPAN vol. 1998, no. 13, 30 November 1998 (1998-11-30) -& JP 10 207340 A (SONY CORP), 7 August 1998 (1998-08-07)**
• **PATENT ABSTRACTS OF JAPAN vol. 2000, no. 25, 12 April 2001 (2001-04-12) -& JP 2001 212240 A (SKY COM:KK), 7 August 2001 (2001-08-07)**

## Description

The invention relates to an audio reproduction apparatus comprising:

- input means for inputting an input audio signal;
- an output for outputting an output audio signal derived from the input audio signal;
- a cost input for inputting a mathematical cost derived from a measurement, which measurement is user-influenceable; and
- a conditioning unit, capable of delivering the output audio signal in dependence of the mathematical cost.

The invention also relates to an audio feedback system comprising:

- an audio source;
- a measurement device arranged to deliver a measurement which is user-influenceable;
- a mathematical cost calculation unit, arranged to derive a mathematical cost from the measurement;
- a sound production device; and
- a conditioning unit arranged to receive an input audio signal from the audio source, to receive the mathematical cost, and to deliver to the sound production device an output audio signal derived from the input audio signal, in dependence of the mathematical cost.

The invention also relates to a method of deriving an output audio signal from an input audio signal in dependence of a mathematical cost derived from a measurement which is user-influenceable.

The invention also relates to a computer program for execution by a processor, describing above mentioned method.

The invention also relates to a data carrier storing the computer program.

An embodiment of such an audio reproduction apparatus is known from US-A-4,788,983. Patents Abstract of Japan for JP 10 207 340A discloses a system for generating a virtual space with audio and visual inputs. US 6 195 434B1 discloses system for generating a virtual sound source at a given position relative to the user. US 4 110 918A discloses a modular biofeedback training system. The known apparatus from US-A-4,788,983 is designed for use by a person performing a sports activity, who wants to listen to music. The known apparatus contains a conditioning unit capable of transmitting an input audio signal from a walkman as an output audio signal to headphones. The conditioning unit also receives a mathematical cost signal from a heart rate measurement device. A user specifies according to e.g. his age or sex a safe window of heart rate which he wants to use during his training. If his heart rate is too low, he is clearly not getting enough exercise. On the other hand, if his heart rate is too high, his exercise might be unhealthy. The conditioning unit transmits the input audio signal only if the measured heart rate is within the desired window, and otherwise no sound is sent to the headphones.

It is a disadvantage of the known apparatus that such a crude regulation of the output audio signal is not user friendly. E.g. if the window is narrow, it is difficult for a user to judge whether he has lost his music because he is running too slow or too fast.

It is an object of the invention to provide an audio reproduction apparatus of the kind described in the opening paragraph, which is relatively versatile as far as the regulation of the output signal is concerned.

The object is realized in that the conditioning unit comprises an audio processing means arranged to process the input audio signal to derive the output audio signal with a reproduction quality in dependence of the mathematical cost. The conditioning unit of the known audio reproduction apparatus contains elements which only implement a switch function. In case the heart rate is outside the window, no signal is sent to the headphones. This is not very desirable. If the user is training just a little too soft, he will hear absolutely no sound. Rather than to be motivating to start running harder again, this can be very demotivating for certain users. It is desirable that there is a gradual change, so that the user can underachieve during a certain period and still have music. Furthermore heart rate measurements are not always reliable, e.g. if a signal of a nearby second user is picked up. The user is then punished for something out of his control. The audio reproduction apparatus according to the present invention is arranged to offer the user many more versatile strategies of responding to his sports activity, embodied as strategies of calculating the mathematical cost. The apparatus according to the invention is also arranged to provide more versatile output audio presentation strategies. Instead of just switching off the output audio signal, the apparatus of the invention offers options of changing the reproduction quality of the output audio signal. This is a physical measurable and determinable correlate to the perceptible psycho-acoustical quality of the sound. E.g., the audio reproduction apparatus may be able to gradually lower the sound amplitude, leading to less intelligible music. Alternatively, if stereo is present an underachieving user can be punished in that the audio reproduction apparatus delivers mono instead of stereo, in which case the number of independent output signals is a reproduction quality measure of the perceived psycho-acoustical quality.

In an embodiment, the reproduction quality comprises a three-dimensional position of a virtual sound source, the audio processing means being able to simulate the virtual sound source by means of the output audio signal. Of the set of all audio-processing functions which can be applied to obtain some psycho-acoustical quality, some realize the audio positioning of a virtual sound source in three-dimensional space around the head of a user. This is especially interesting for the user traveling a distance -real or virtual- such as a jogger or somebody on a bicycle hometrainer. If he instructs the apparatus that he should run at a certain speed, he should be at a certain position -real or virtual- at a certain time. The audio reproduction apparatus can position the virtual sound source by sending an appropriate audio signal to a left and a right loudspeaker of the headphones, the virtual sound source being e.g. two virtual loudspeakers at a distance of 1 meters in front of the user. If the user runs too slow the virtual loudspeakers move away from him, and this can be simulated if desired by making the sound emerging from the virtual loudspeakers become ever less audible. The user can catch up with the loudspeakers by running faster. By adding synthetic reverberations other three-dimensional audio position quality measurements can be influenced, such as the illusion of a wall in front of the user.

In a modification of the embodiment, the audio processing means comprise a filter arranged to simulate the position of virtual sound source by deriving the output audio signal by filtering the input audio signal with a user dependent head related transfer function (HRTF). By means of an HRTF a sound source such as a virtual loudspeaker can be accurately positioned. The input audio signal for the left resp. the right headphone loudspeaker is filtered by a respective HRTF, simulating the path through a virtual room of sound from an actual loudspeaker at a position in the room to the respective ear of the user.

In another modification of the embodiment or a further variation on the foregoing modification, the audio processing means comprise an audio processing unit arranged to simulate the position of the virtual sound source by changing a property of the output audio signal selected from signal amplitude and added reverberation. Both properties are simple signal processing functions to realize the illusion of sound with a specific three-dimensional audio position quality.

The audio processing means can also be arranged to derive a second output audio signal, together with the output audio signal constituting a stereo audio signal, the audio processing means being arranged to derive the stereo audio signal from the input audio signal with a specified stereo quality dependent on the mathematical cost. Signal processing functions influencing the stereo quality are e.g. the following:

- the virtual loudspeakers are moved closer to each other if the user underachieves;
- the audio signals for the left and right virtual loudspeakers are made more similar if the user underachieves; or
- one of the virtual loudspeakers disappears if the user underachieves.

The last option can also be implemented as a -gradual or brusque- switch between multichannel surround such as Dolby 5.1 and 2 channel stereo. Varying the stereo quality is advantageous given the widespread presence of stereo sound.

It is advantageous if the reproduction quality comprises a specification of the distribution of frequencies of the output audio signal. By changing the frequency content of the output audio signal, the audio processing means can simulate other effects. E.g. an underachiever is punished by removing the bass of the audio signal. The three-dimensional position of a virtual sound source can be influenced by such an audio processing function too. E.g. the audio processing means may be arranged to remove high frequencies as if the sound has to travel a long way through a thick fog, or resides at a depth in a virtual wall.

It is also advantageous if the audio reproduction apparatus comprises a first quality calculation unit for determining the reproduction quality for use in the subsequent derivation of the output audio signal by the audio processing means. The reproduction quality is a property of the output audio signal. The audio reproduction apparatus could measure it on the output audio signal, but then the input audio signal first has to be processed by an a priori unknown processing algorithm. A correlate reproduction quality can be determined by the first quality calculation unit and send to the audio processing means which applies a corresponding processing function. E.g. changing the angle of two virtual loudspeakers has an influence on the stereo quality, and it is in general not necessary to specify what numerical stereo quality a user experiences. If more accuracy is desired, a specific angle function can be stored in a memory, e.g. based upon user panel tests. It is also possible that the user specifies a relation between e.g. his running speed and the angle himself, or chooses between a number of prestored functions, some of which change the angle slowly and other quickly.

Alternatively or additionally it is advantageous if the audio reproduction apparatus comprises quality measuring means for measuring an output quality measure of the output audio signal, and comprises parameter value calculation means for calculating a parameter value, for use in the subsequent derivation of the output audio signal by the audio processing means. If the quality of the output signal is measured, it can be fed back for changing the processing on the input audio signal for future times. Such a feedback control loop obtains the desired reproduction quality after some tuning time. The parameter value calculation means can take an incompatibility between the result of the reproduction quality measurement and the desired quality measurement into account. The parameter value is changed accordingly, steering the processing function until it obtains the desired audio output signal reproduction quality.

In another embodiment a mathematical cost calculation unit is comprised which is arranged to derive the mathematical cost from the measurement receivable from a measurement device. A mathematical cost can be derived from any kind of apparatus, e.g. a random generator if the audio reproduction apparatus is used in a competitive game. Typically the mathematical cost may be determined on the basis of a measurement which the user can influence, such as his running speed, heart rate, etc. A measurement device could send the mathematical cost directly to the apparatus, e.g. as a coded number. Typically however the audio reproduction apparatus may contain the new functionality, so that it can be used with an off-the-shelf measurement device.

In a modification of the previous embodiment, the mathematical cost calculation unit is arranged to derive the mathematical cost based on a difference between the measurement and a chosen value. E.g., the user sets this chosen value being his desired running speed as 10 km/h, or his desired heart rate as 180 bpm. The quality is then e.g. determined as the actual running speed minus 10 km/h. The faster he runs, the more the reproduction quality changes. Or in an alternative version, if he runs a little harder nothing happens, and if he runs a lot harder the audio processing means are arranged so that the reproduction quality starts changing gradually depending on the amount of time he is running harder than the chosen value.

In an alternative modification of the previous embodiment or in addition to the previous modification, the mathematical cost calculation unit is arranged to derive the mathematical cost from a biometric measurement. Engineering quality audio systems and biometric measurements are totally unrelated fields of technology. Apparently, nobody sees a need of combining them. Biometric measurement systems are usually designed by engineers who work in close cooperation with physicians, and priorities in this field are exactness and robustness of the measurements and safety. The quality of audio reproduction is more an artistic matter of taste. This has led to the fact that biometric measurements are usually displayed on numeric displays. An exception is the beeps of a medical monitor, but the audio functionality of such monitors is designed for simplicity rather than artistic reproduction quality. There is a need for user-friendly feedback of biometric data while training, since a user does not like to watch a display continuously while training. Sound however when present automatically enters the user's ear.

The audio feedback system is characterized in that the conditioning unit comprises an audio processing means arranged to process the input audio signal for the derivation of the output audio signal with a reproduction quality in dependence of the mathematical cost. It is advantageous to produce the system as a whole since then all components can be realized as being optimally tuned to each other.

The method of deriving an output audio signal from the input audio signal in dependence of the mathematical cost derived from a measurement which is user-influenceable is characterized in that the output signal is derived with a specified reproduction quality dependent on the mathematical cost.

These and other aspects of the audio reproduction apparatus, the method, the audio feedback system, the computer program and the data carrier according to the invention will be apparent from and elucidated with reference to the implementations and embodiments described hereinafter, and with reference to the accompanying drawings, which serve merely as non limiting illustrations.

In the drawings :

Fig. 1 schematically shows an application of the audio reproduction apparatus;
Fig. 2 schematically shows an embodiment of the audio reproduction apparatus;
Fig. 3 schematically shows an embodiment of the audio processing means of the audio reproduction apparatus;
Fig. 4 schematically shows an embodiment of the audio feedback system;
Fig. 5 schematically shows an embodiment of the data carrier;
Fig. 6a and b schematically show a respective example of a mathematical cost function;
Fig. 7 schematically shows an example specification of a position quality as a function of mathematical cost; and
Fig. 8 schematically shows an example of a frequency spectrum of an output audio signal output by the audio reproduction apparatus.

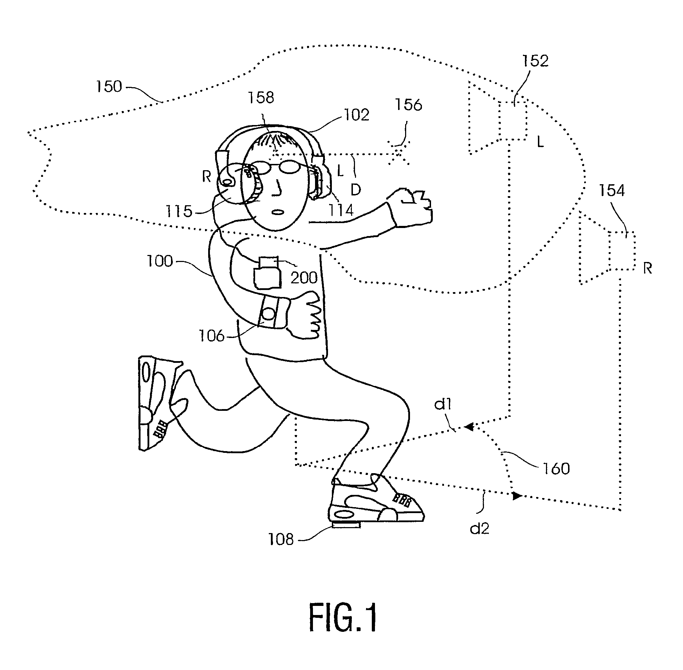

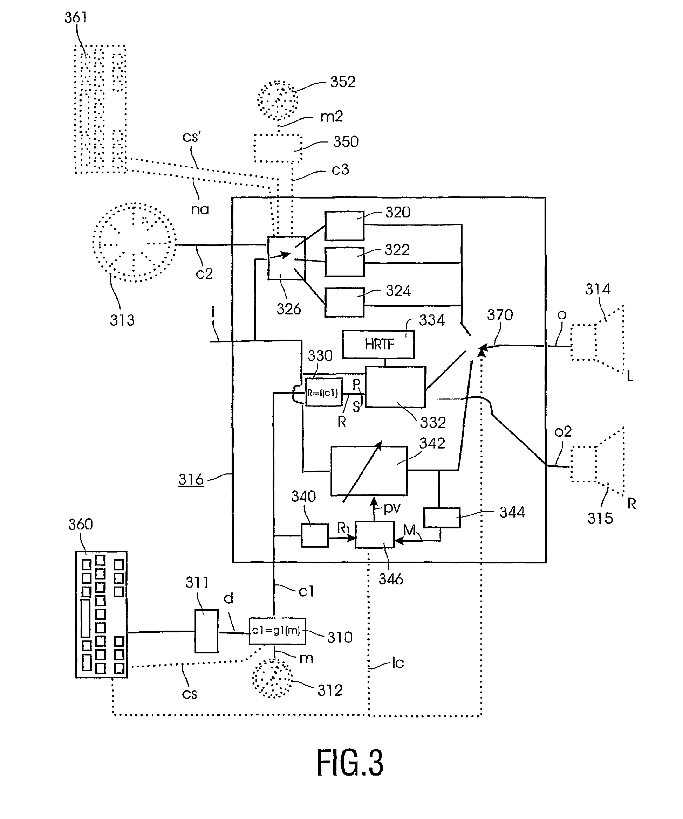

In these Figures elements drawn dashed are virtual in Fig. 1, and optional in the other Figures, depending on the desired embodiment. Not all elements present in the illustrative embodiment of the audio processing means in Fig. 3 need be present in an alternative embodiment.

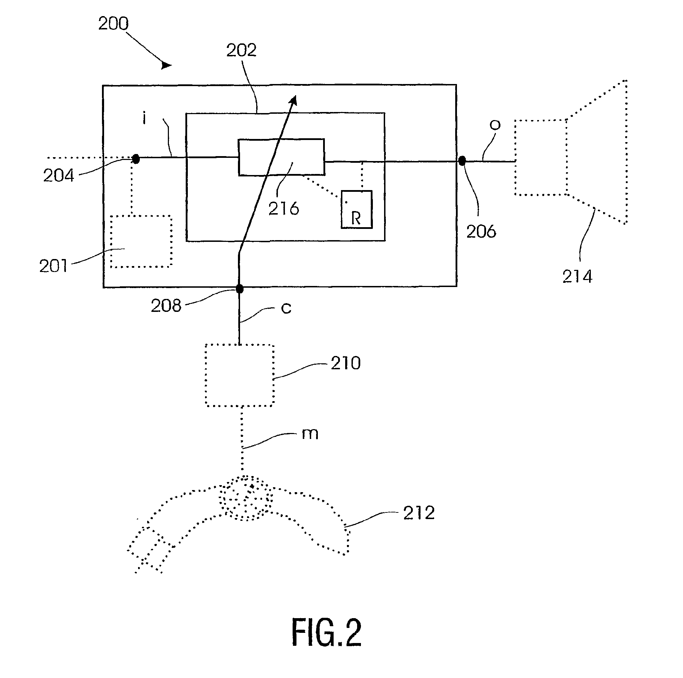

Fig. 1 shows a user 100 of the audio reproduction apparatus 200 according to the invention, who is jogging. He could e.g. also be rowing on an indoors rowing machine. While doing his sports activity he is listening to music coming as an output audio signal o-see Fig. 2- from the audio reproduction apparatus 200, being e.g. a portable audio player such as an MP3 player, and reproduced by the left and right headphone loudspeaker 114 and 115 of a sound production device 102, typically being headphones. The reproduction quality R of the music is changed by the audio reproduction apparatus 200 in dependence of the sports performance of the user 100, e.g. varying between Dolby 5.1 and mono without bass frequencies. E.g. if he runs too slow, he is penalized with music of bad reproduction quality R. His performance can be measured by at least one of various sensors. E.g. a pace meter 108 connected to his sports shoe or another measurement device like a heart rate meter 106 delivers a measurement signal m. For the heart rate meter 106 this measurement can be e.g. a PQRST complex of an electrocardiogram, a time sequence of pulses, or a number representing the heart rate. The audio reproduction apparatus 200 converts this measurement m to a reproduction quality R.

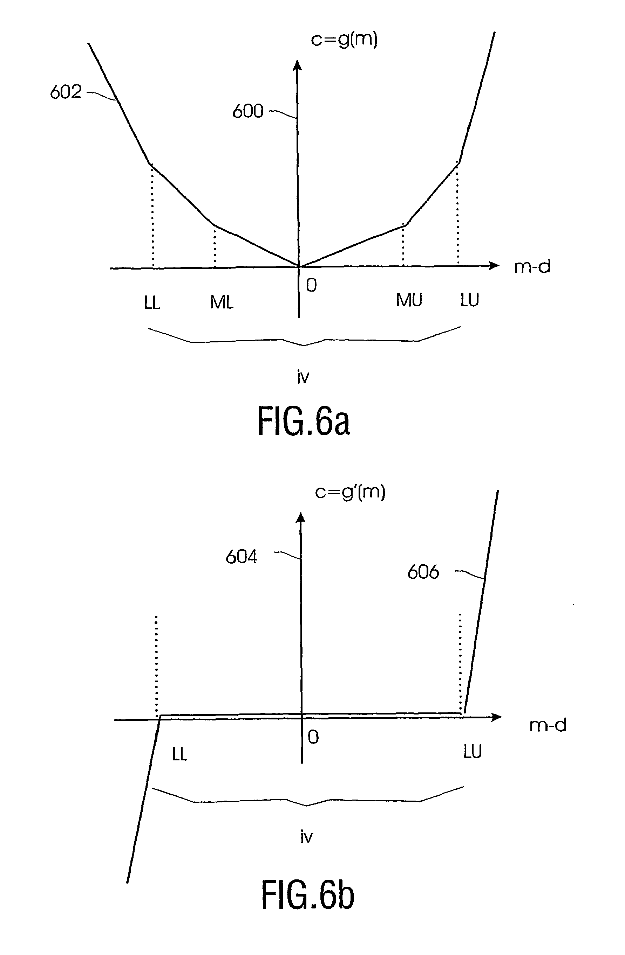

In a simple variant of the audio reproduction apparatus 200, the audio reproduction strategy is fixed, and the user 100 can only specify the way in which the measurement is transformed into a mathematical cost c. Note that for clarity reasons the examples are described for an embodiment in which all the mathematical transformations are realized as software algorithms running on a processor, but dedicated hardware circuitry could also be used. E.g. the user 100 can specify an interval iv in which his heart rate should lie such as [LL, LU] in Fig. 6a. A mathematical cost function 602 is shown in a coordinate system 600 with on the x-axis the heart rate measurement m minus a chosen value d and on the y-axis the mathematical cost c. This chosen value d is set by the user as the target heart rate for his training, e.g. 180 bpm. The interval [LL, LU] can be symmetrical or asymmetrical around the chosen value d. The mathematical cost function can be fixed in the hardware of the audio reproduction apparatus 200 of Fig. 2, or the user can specify by means of user interface means 311 of Fig. 3 how the mathematical cost changes with m-d. E.g. as in Fig. 6a up to the marker values ML and MU the mathematical cost changes linearly with a small slope, while between the marker values and the limits of the interval iv the mathematical cost slopes more steeply, and outside the interval iv the mathematical cost increases very steeply. In an alternative mathematical cost function 606 specification shown in Fig. 6b, the mathematical cost is non-zero and linearly changing only outside the training interval iv. E.g. in Fig. 6b the user 100 has designed a cost function with negative values if he is running too slow. A negative cost can then during reproduction easily be mapped e.g. as a negative angle $\alpha$ of of a virtual sound source, and a positive cost corresponding to speeds which are too high as a positive angle $\alpha$. In this way both cases can be easily discriminated. The user has freedom in designing the cost function by choosing e.g. training interval limits, quickness of change of the cost -which can be translated into quickness of change of audio reproduction quality R-, whether only measurements below the chosen value d are leading to nonzero cost, etc.

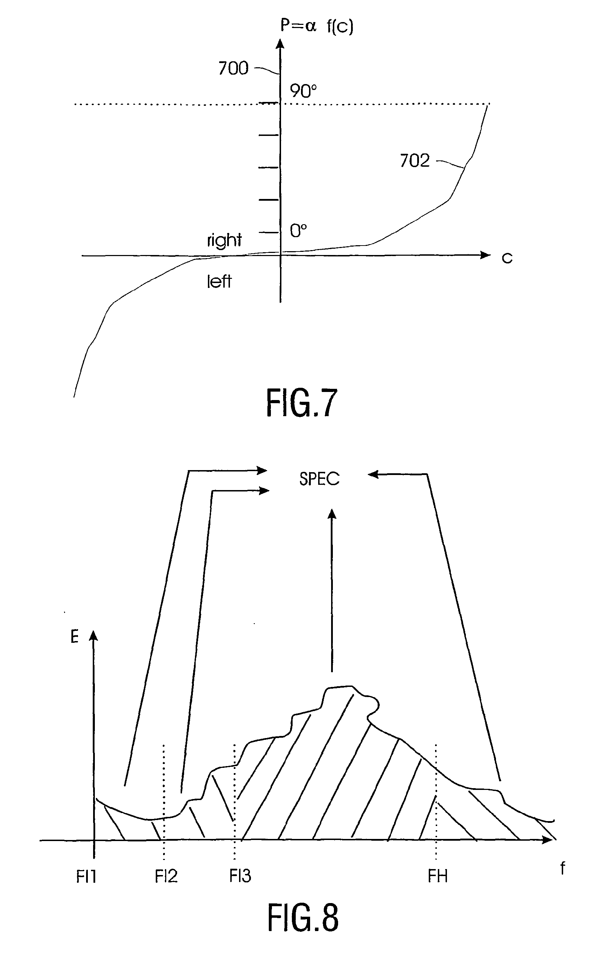

The user interface means 311 are e.g. software running on a processor, which requests the user to type numerical values of marker values and slopes, or which allows the user to draw the mathematical cost function 602 graphically. The fixed audio reproduction strategy is e.g. the one illustrated in Fig. 7. Here the reproduction quality R is embodied as what is called in the rest of the text a position reproduction quality P, which is any specification of a physical parameter of the output audio signal o resulting in the perception that the output audio signal comes from a position of a virtual sound source. E.g. the virtual sound source can be perceived close to a user's head or far away, or in Fig. 7 it is an angle $\alpha$ of a virtual sound source around a user's head. If the user 100 is running with the desired target speed, the mathematical cost c is zero and the angle $\alpha$ is also zero degrees, i.e. the virtual sound source is right in front of the user 100. If the runner runs too slow or fast, the mathematical cost c with a specification like in Fig. 6b decreases resp. increases, and the virtual sound source shifts to the left resp. right side of the user 100. The sound source can stay behind the user until the user 100 runs with the desired speed of the chosen value d again or runs with the desired speed again for a certain amount of time. Alternatively, the sound source can also start behind the user 100, being an annoying motivation to run harder.

In more advanced variants of the audio reproduction apparatus 200, the user 100 can also specify the strategy for changing the reproduction quality as a function of the mathematical cost c. He can program a first quality calculation unit 330 in Fig. 3 which outputs e.g. as a stereo quality S an angle 160 between a first virtual sound source 152 and a second virtual sound source 154 generated by the output audio signal o and a second output audio signal o2, as a linear function of the mathematical cost c. Or he can select an alternative or additional audio processing function, which e.g. adds an amount of reverberation as a function of the mathematical cost c to simulate a distance of a virtual loudspeaker in a virtual room.

Another example application of the audio reproduction apparatus 200 is the prevention of repetitive strain injury (RSI) or inactivity. In this case user 100 sits e.g. in front of a personal computer (PC) or on a couch in front of a television (TV). The sound production device 102 is e.g. a loudspeaker connected to the PC or television. The chosen value d is the amount of time the user 100 wants to work or watch TV before taking a break. The mathematical cost c is e.g. determined by the amount of time t elapsed since a starting time t0 minus the chosen value d, being the allowable time to work or watch TV continuously:

$$c = (t - t_0) - d \ \text{ if } t - t_0 > d \ ;$$

$$c = 0 \ \text{ if } t - t_0 < d \qquad\qquad [1]$$

With two loudspeakers of a TV or PC, a virtual sound source position can be simulated.

Fig. 2 schematically shows an embodiment of the audio reproduction apparatus 200 in its basic form. An input audio signal i comes in via an input means 204 from e.g. a portable MP3 player or the sound card of a PC . The input audio signal i can come form outside or inside the audio reproduction apparatus 200. In the latter case the audio reproduction apparatus 200 may comprise e.g. a CD player unit or any other internal audio source 201. The input audio signal i can be mono or multichannel audio. There is also a cost input 208 for receiving a mathematical cost c from a mathematical cost calculation unit 210, which is arranged to derive the mathematical cost from a measurement made by a measurement device 212. The mathematical cost calculation unit 210 can be incorporated in the audio reproduction apparatus 200 or can be separate, e.g. in the measurement device 212. The measurement device 212 is typically not incorporated in the audio reproduction apparatus 200, although it could be, in case it is e.g. a clock. The audio reproduction apparatus 200 contains a conditioning unit 202, which contains an audio processing means 216 arranged to process the input audio signal i to derive the output audio signal o with a reproduction quality R in dependence of the mathematical cost c. The output audio signal o goes to an output 206, to which a loudspeaker 214 can be connected. The audio processing means 216 can just perform a single parametric function leading to an output audio signal o of variable reproduction quality and hence perceptible psycho-acoustical quality, or multiple audio processing algorithms can be applied alternatively or simultaneously as in Fig. 3.

Fig. 3 schematically shows an audio processing means 316, being an embodiment of the audio processing means 216 of the audio reproduction apparatus 200. In the audio processing means 316 a number of processing units are shown purely for explaining various features of the audio reproduction apparatus 200, and it should be clear that other combinations are possible. The audio processing means 316 is arranged to supply an output audio signal o to a first loudspeaker 314 and if required a second output audio signal o2 to a second loudspeaker 315.

For many audio processing algorithms, the reproduction quality R can be set in advance, and a subsequent audio processing is chosen depending on the reproduction quality R. E.g. the reproduction quality R can be a parameter of an audio processing algorithm, as in the case where the amplitude of the output audio signal is set. This can be realized with a variable gain amplifier. In other cases, user panel tests or the preferences of the actual user of the audio reproduction apparatus 200 can be used to select an appropriate processing algorithm, e.g. the first, second or third processing algorithm, 320, 322 resp. 324. In the example embodiment of Fig. 3, a third mathematical cost c3 from a second measurement device 352 goes to an algorithm selector 326, which e.g. contains a table of intervals. If the third mathematical cost c3 falls within a first interval, the first processing algorithm 320 is selected, etc. Such a configuration makes it possible to switch to entirely different algorithms dependent on the value of the third mathematical cost c3. E.g., the first algorithm may change the angle 160 between two virtual loudspeakers, depending on where in the first interval the third mathematical cost c3 falls. If the third mathematical cost c3 becomes so high that it falls outside the first interval and in a second interval, the second processing algorithm 322 is selected, which e.g. changes the amplitude of the signals from the virtual loudspeakers, or both the angle 160 between them and the signal amplitudes. Another example in which the reproduction quality R is set prior to the processing is the setting of an angular position on a sphere around the user's 100 head of a virtual sound source by means of a head related transfer function HRTF. E.g., when the user 100 wears headphones, the input audio signal i can be simulated to come from a virtual sound source position, by filtering it by means of filter 332 using a specific first HRTF to obtain the output audio signal o for the left headphone loudspeaker 114 and using a specific second HRTF to obtain the second output audio signal o2 for the right headphone loudspeaker 115. Both HRTFs are dependent on the required position of the virtual sound source- e.g. specified as two angles on a unit sphere- and can be fetched from a memory 334 containing HRTFs for a number of different positions. A first quality calculation unit 330 determines the reproduction quality R needed for further audio processing. E.g. in the above described case the first quality calculation unit 330 calculates an angle $\alpha$ of the virtual sound source, used for fetching the HRTFs, as a linear function of a first mathematical cost cl. The first mathematical cost cl is derived from a measurement device 312 by a mathematical cost calculation unit 310, which e.g. evaluates a function like the one in Fig. 6a. Details on measuring HRTFs can be found in patent WO 01/49066 and paper "F.L. Wightman and D.J. Kistler: Headphone simulation of free field listening. I: Stimulus synthesis. Journal of the Acoustical Soc. of America 85 no. 2, Feb. 1989, pp. 858-867.".

In other cases, the reproduction quality R has to be measured on the output audio signal o itself, e.g. because the relation between the reproduction quality R of the output audio signal o and the particular processing is too complex to formulate or unknown. In this case feedback can be used to select the right processing algorithm or the right parameter for a parametric processing algorithm. Quality measuring means 344 measure an output quality measure M of the output audio signal o. The output quality measure M and a desired reproduction quality R from a second quality calculation unit 340 are fed to a parameter value calculation means 346. From these two parameters, a parameter value pv is calculated for steering subsequent processing by an audio processing unit, which selects a particular processing algorithm or changes a parameter of a parametric algorithm. This can be done by any technique known from control theory. E.g. An update parameter value pv can be calculated as in equation [2]:

$$pv = \delta(M - R) \qquad\qquad [2]$$

Actually parameter value pv can be any function of M and R, if necessary also taking into account that the output quality measure M is a different function of the desired psycho-acoustical quality than the reproduction quality R.

The required implicit functionality between the output quality measure M and the reproduction quality R derived from the first mathematical cost c1, can be specified by user 100. With user input means 360, e.g. a key board, a touch sensitive panel, or a turning knob and user interface means 311, the user 100 can specify a number of desired measurement values d, which are converted to corresponding first mathematical costs c1 and reproduction qualities R. Instead of inputting desired measurement values d, the user 100 can also input desired mathematical costs cs. During this learning stage, for each reproduction quality R a number of processing algorithms with corresponding output quality measures M is scanned. When the output quality measure M corresponding to a psycho-acoustical quality as desired by the user 100 is reached, the user 100 can indicate this to the parameter value calculation means 346 via a learning control connection lc -wired or wireless. The parameter value calculation means 346 can then store the pair reproduction quality R and parameter value pv, so that during operation, the feedback is no longer needed, but rather that from a reproduction quality R corresponding to a measurement m, the correct parameter value pv can be sent to an audio processing unit 342. The learning control connection lc can also be used to specify which of the available processing should be used for obtaining an output audio signal o with the desired reproduction quality R, by setting an output selector 370.

Another example of putting user preferences in the audio processing means 316 is illustrated with a second user input means 361. In this example, the user 100 can directly enter a second desired mathematical cost cs' and a corresponding desired processing algorithm selection na into the algorithm selector 326.

Note that in many cases the exact perceived psycho-acoustical quality, e.g. the exact position of a virtual sound source is not important, but only that the psycho-acoustical quality changes monotonically. This relaxes the requirements on the reproduction strategy. Any mapping of virtual sound source angle to mathematical cost e.g. might already be sufficient.

Many processing algorithms can be designed to create some perceptible psycho-acoustical quality corresponding to a reproduction quality R characterizing a selected algorithm. E.g. a reproduction of bass frequencies can be changed in dependence of the mathematical cost. As shown in Fig. 8, as a particular reproduction quality R or part of a reproduction quality R, a specification SPEC can be calculated reflecting the spectral content of the output audio signal o. One example of the specification SPEC is a frequency below which there is substantially no sound energy present, e.g. a first low frequency FL1 or a second low frequency FL2. E.g. if the user runs at nearly the desired speed, bass frequencies are reproduced all the way down to the first low frequency FL1. However if he runs to slow, he looses the bass frequencies between the first low frequency FL1 and the second low frequency FL2. Another example of the specification SPEC is the percentage of energy in bass range [FL1, FL2] compared to the energy in range [FL3, FH]. Any equalization strategy can be employed as a function of the cost c, e.g. the amount of trebble may be a function of the cost c.

An interesting algorithm sets the mathematical cost function by means of a three-dimensional bubble 150 around the user's 100 head. If he runs too slow, the distance between the virtual position of his head 158 and a mark point 156 in the bubble increases, which leads to an increased mathematical cost c, and a decreased reproduction quality R. The reproduction quality R can also change in dependence of whether the user 100 is inside the bubble 150 or not, which gives him a training tolerance. The virtual movement of the bubble 150 compared to the running of the user could even keep track of whether the user was waiting for traffic lights, this situation being identified e.g. when he pushes a button. When inside the bubble 150, the sound could e.g. sound as if the user 100 is in a particular room, by selecting HRTFs corresponding to that room, whereas outside the bubble 150, the sound sounds dull.

The distance of a virtual sound source can also be simulated. The audio-processing unit 342 can e.g. simulate the distance of the virtual sound source by changing the amplitude of the output audio signal o and second output audio signal o2. Or reverberation can be added. In a room if a sound source is nearby, there is little reverberation, whereas if the sound source is far, there is a greater proportion of reverberation. Additionally or alternatively, virtual corridors can be generated, on which the virtual sound source reflects its sound.

A number of options are also possible for changing a stereo quality S of a stereo audio signal. E.g. an angle 160 between a first virtual sound source 152 and a second virtual sound source 154 can be diminished as the mathematical cost c goes up, resulting in a lower stereo quality S. Or the first and second output audio signal o resp. o2 can be made more similar. Or there can be a hard switch from stereo to mono. Under stereo signal should also be understood multichannel audio, and a corresponding stereo quality S is e.g. the number of channels.

An example of a gradual change between stereo and mono is realized by calculating changed stereo signals L' and R'according to the following equations:

$$L' = (1+a)\frac{L}{2} + (1-a)\frac{R}{2}$$

$$R' = (1-a)\frac{L}{2} - (1+a)\frac{R}{2} \qquad [3]$$

By changing the value of a parameter a between 0 and 1, a change between mono and stereo is effected.

The measurement device 312 and second measurement device 352 can be anything, e.g. a clock or a G.P.S. sensor indicating the position of the user 100. In particular it could be a biometric measurement device, such as a pace meter connected to a running shoe, a chest strap or ear-lobe heart rate meter 106, a thermometer, etc. These measuring devices are interesting when used for sports performance measurement. The measurement device 312 could also be incorporated in a professional training apparatus, e.g. a rolling belt for indoors jogging. The style of running -particularly if unhealthy- could also be fed back.

In a game application, a second mathematical cost c2 can be set by a cost determining means 313, e.g. a random generator. Depending on his luck, the user's 100 mathematical cost is set back to the second mathematical cost c2 and he has to run harder to re-achieve the level of the third mathematical cost c3, as determined by a second mathematical cost calculation unit 350 from the second measurement device 352.

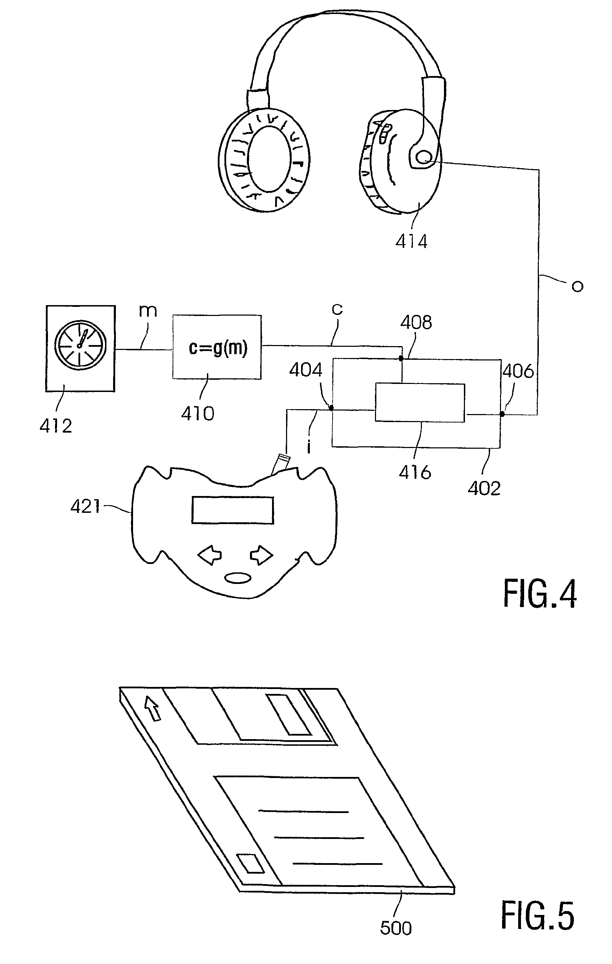

Fig. 4 schematically shows an embodiment of the audio feedback system. An audio source 421 delivers an input audio signal i to a conditioning unit 402. The audio source 421 can be e.g. a portable audio player or an audio distribution server in a fitness center. A measurement device 412 performs a measurement m, which is converted by a mathematical cost calculation unit 410 to a mathematical cost c, which is also input in the conditioning unit 402. The conditioning unit 402 contains an audio processing means 416, which are arranged to process the input audio signal to obtain an output signal o of a reproduction quality R dependent on the mathematical cost c, which is sent to a sound production means 414, e.g. a pair of headphones or the loudspeakers of a television. The measurement device 412, mathematical cost calculation unit 410, conditioning unit 402, audio source 421, and sound production means 414 can be realized separately or in any combination. E.g., typically an audio reproduction apparatus may contain the conditioning unit 402, audio source 421 and mathematical cost calculation unit 410.

A head tracker may be present, so that the positions of virtual sound sources are corrected for movements of the user's 100 head. Also means may be present -e.g. a microphone- to pick up certain sounds from the environment and mix them with the signal for the sound production device 414 for improved safety.

Specification of e.g. the cost function may instead of being done by the user 100 come over a channel -such as e.g. internet- through an interface, from a second person, e.g. a personal trainer. Or the specification may be done at a different time by the user 100, e.g. on his PC. E.g., he can make a training schedule for the whole month, which can be downloaded e.g. wirelessly to the audio reproduction apparatus. Parameters of functions and functions may even be downloaded from e.g. internet, and e.g. shared between users who want similar training sessions. Specifications made during training and stored in a memory, may also be downloaded through the interface to the computer for further analysis, e.g. training improvement.

It should be noted that the above-mentioned embodiments illustrate rather than limit the invention and that those skilled in the art are able to design alternatives, without departing from the scope of the claims. Apart from combinations of elements of the invention as combined in the claims, other combinations of the elements within the scope of the invention as perceived by one skilled in the art are covered by the invention. Any combination of elements can be realized in a single dedicated element. Any reference sign between parentheses in the claim is not intended for limiting the claim. The word "comprising" does not exclude the presence of elements or aspects not listed in a claim. The word "a" or "an" preceding an element does not exclude the presence of a plurality of such elements.

The invention can be implemented by means of hardware or by means of software running on a computer, and previously stored on a data carrier or transmitted over a signal transmission system.

## Claims

1. An audio reproduction apparatus (200) comprising:

    - input means (204) for inputting an input audio signal (i);
    - an output (206) for outputting an output audio signal (o) derived from the input audio signal (i);
    - a cost input (208) for inputting a mathematical cost (c) derived from a measurement (m), which measurement (m) is user-influenceable; and

- a conditioning unit (202), capable of delivering the output audio signal (o) in dependence of the mathematical cost (c),

**characterized in that** the conditioning unit (202) comprises an audio processing means (216, 316) arranged to process the input audio signal (i) to derive the output audio signal (o) with a reproduction quality (R) in dependence of the mathematical cost (c); and **characterized in that** the reproduction quality comprises at least one of:

(a) a specified stereo quality of the output audio signal being a stereo audio signal;
(b) a number of independent output signals;
(c) a specification of a distribution of frequencies of the output audio signal; and
(d) an amplitude of the output audio signal.

2. An audio reproduction apparatus (200) as claimed in claim 1, wherein the reproduction quality (R) comprises a three-dimensional position of a virtual sound source (152), the audio processing means (216, 316) being able to simulate the virtual sound source (152) by means of the output audio signal (o).

3. An audio reproduction apparatus (200) as claimed in claim 2, wherein the audio processing means (316) comprises a filter (332) arranged to simulate the position of the virtual sound source (152) by deriving the output audio signal (o) by filtering the input audio signal (i) with a user dependent head related transfer function (HRTF).

4. An audio reproduction apparatus (200) as claimed in claim 2, wherein the audio processing means (316) comprises an audio processing unit (342) arranged to simulate the position of the virtual sound source (152) by changing a property of the output audio signal (o) selected from signal amplitude and added reverberation.

5. An audio reproduction apparatus (200) as claimed in claim 1, comprising a first quality calculation unit (330) for determining the reproduction quality (R) for use in the subsequent derivation of the output audio signal (o) by the audio processing means (216).

6. An audio reproduction apparatus (200) as claimed in claim 1, comprising quality measuring means (344) for measuring an output quality measure (M) of the output audio signal (o), and comprising parameter value calculation means (346) for calculating a parameter value (pv), for use in the subsequent derivation of the output audio signal (o) by the audio processing means (216).

7. An audio reproduction apparatus (200) as claimed in claim 1, wherein a mathematical cost calculation unit (310) is comprised which is arranged to derive the mathematical cost (c) from the measurement (m) receivable from a measurement device (312).

8. An audio reproduction apparatus (200) as claimed in claim 7, wherein the mathematical cost calculation unit (310) is arranged to derive the mathematical cost (c) based on a difference between the measurement (m) and a chosen value (d).

9. An audio reproduction apparatus (200) as claimed in claim 7, wherein the mathematical cost calculation unit (310) is arranged to derive the mathematical cost (c) from a biometric measurement.

10. An audio feedback system comprising:

- an audio source (421);
- a measurement device (412) arranged to deliver a measurement (m) which is user-influenceable;
- a mathematical cost calculation unit (410), arranged to derive a mathematical cost (c) from the measurement (m);
- a sound production device (414); and
- a conditioning unit (402) arranged to receive an input audio signal (i) from the audio source (421), to receive the mathematical cost (c), and to deliver to the sound production device (414) an output audio signal (o) derived from the input audio signal (i), in dependence of the mathematical cost (c),

**characterized in that** the conditioning unit (402) comprises an audio processing means (416) arranged to process the input audio signal (i) to derive the output audio signal (o) with a reproduction quality (R) in dependence of the mathematical cost (c); and **characterized in that** the reproduction quality comprises at least one of:

(a) a specified stereo quality of the output audio signal being a stereo audio signal;
(b) a number of independent output signals;
(c) a specification of a distribution of frequencies of the output audio signal;
(d) an amplitude of the output audio signal.

11. A method of deriving an output audio signal (o) from an input audio signal (i) in dependence of a mathematical cost (c) derived from a measurement (m) which is user-influenceable, **characterized in that** the output signal (o) is derived with a specified reproduction quality (R) dependent on the mathematical cost (c); and **characterized in that** the reproduction quality comprises at least one of:

(a) a specified stereo quality of the output audio signal being a stereo audio signal;
(b) a number of independent output signals;
(c) a specification of a distribution of frequencies of the output audio signal;
(d) an amplitude of the output audio signal.

12. A computer program for execution by a processor, describing the method of claim 11.

13. A data carrier (500) storing the computer program of claim 12.

**Patentansprüche**

1. Audiowiedergabeanordnung (200), die Folgendes umfasst:

- Eingabemittel (204) zum Eingeben eines Eingangs-Audiosignals (i);
- einen Ausgang (206) zum Liefern eines aus dem Eingangs-Audiosignal (i) hergeleiteten Ausgangs-Audiosignals (o);
- einen Preiseingang (208) zum Eingeben eines aus einer Messung (m) hergeleiteten mathematischen Preises (c), wobei diese Messung (m) vom Benutzer beeinflussbar ist; und
- eine Aufbereitungseinheit (202), die imstande ist, in Abhängigkeit von dem mathematischen Pries (c) das Ausgangssignal (o) zu liefern,

**dadurch gekennzeichnet, dass** die Aufbereitungseinheit (202) ein Audioverarbeitungsmittel (216, 316) aufweist, vorgesehen zum Verarbeiten des Eingangs-Audiosignals (i) zum Herleiten des Ausgangs-Audiosignals (o) mit einer Wiedergabequalität (R) in Abhängigkeit von dem mathematischen Preis (c) und **dadurch gekennzeichnet, dass** die Wiedergabequalität wenigstens Folgendes umfasst:

(a) eine festgelegte Stereoqualität des Ausgangs-Audiosignals, d. h. ein Stereo-Audiosignal;
(b) eine Anzahl unabhängiger Ausgangssignale;
(c) eine Spezifikation einer Verteilung von Frequenzen des Ausgangs-Audiosignals; oder
(d) eine Amplitude des Ausgangs-Audiosignals.

2. Audiowiedergabeanordnung (200) nach Anspruch 1, wobei die Wiedergabequalität (R) eine dreidimensionale Position einer virtuellen Schallquelle (152) aufweist, wobei das Audioverarbeitungsmittel (216, 316) imstande ist, mit Hilfe des Ausgangs-Audiosignals (o) die virtuelle Schallquelle (152) zu simulieren.

3. Audiowiedergabeanordnung (200) nach Anspruch 2, wobei das Audioverarbeitungsmittel (316) ein Filter (332) aufweist, vorgesehen zum Simulieren der Position der virtuellen Schallquelle (152) durch Herleitung des Ausgangs-Audiosignals (o) durch Filterung des Eingangs-Audiosignals (i) mit einer benutzerabhängigen, kopfbezogenen Übertragungsfunktion (HRTF).

4. Audiowiedergabeanordnung (200) nach Anspruch 2, wobei das Audioverarbeitungsmittel (316) eine Audioverarbeitungseinheit (342) aufweist, vorgesehen zum Simulieren der Position der virtuellen Schallquelle (152) durch Änderung des Ausgangs-Audiosignals (o), selektiert aus Signalamplitude und hinzugefügtem Nachhall.

5. Audiowiedergabeanordnung (200) nach Anspruch 1, mit einer ersten Qualitätsberechnungseinheit (330) zum Ermitteln der Wiedergabequalität (R) zur Verwendung in der nachfolgenden Herleitung des Ausgangs-Audiosignals (o) durch das Audioverarbeitungsmittel (216).

**6.** Audiowiedergabeanordnung (200) nach Anspruch 1, mit Qualitätsmessmitteln (344) zum Messen eines Ausgangsqualitätsmaßes (M) des Ausgangs-Audiosignals (o), und mit Parameterwertberechnungsmitteln (346) zum Berechnen eines Parameterwertes (pv) zur Verwendung in der nachfolgenden Herleitung des Ausgangs-Audiosignals (o) durch das Audioverarbeitungsmittel (216).

**7.** Audiowiedergabeanordnung (200) nach Anspruch 1, wobei eine Berechnungseinheit (310) für den mathematischen Preis vorhanden ist, und zwar vorgesehen zum Herleiten des mathematischen Preises (c) aus der von einer Messanordnung (312) empfangenen Messung (m).

**8.** Audiowiedergabeanordnung (200) nach Anspruch 7, wobei die Berechnungseinheit (310) für den mathematischen Preis zum Herleiten des mathematischen Preises (c) auf Basis einer Differenz zwischen der Messung (m) und einem gewählten Wert (d) vorgesehen ist.

**9.** Audiowiedergabeanordnung (200) nach Anspruch 7, wobei die Preisberechnungseinheit (310) zum Herleiten des mathematischen Preises (c) aus einer biometrischen Messung vorgesehen ist.

**10.** Audiorückkopplungssystem, das Folgendes umfasst:

- eine Audioquelle (421);
- eine Messanordnung (412) zum Liefern einer Messung (m), die vom Benutzer beeinflussbar ist;
- eine Preisberechnungseinheit, vorgesehen zum Herleiten eines mathematischen Preises (c) aus der Messung (m);
- eine Tonwiedergabeanordnung (414); und
- eine Aufbereitungseinheit (402), vorgesehen zum Empfangen eines Eingangs-Audiosignals (i) aus der Audioquelle (421) zum Empfangen des mathematischen Preises (c) und zum Liefern eines aus dem Eingangs-Audiosignal (i) hergeleiteten Ausgangs-Audiosignals (o) zu der Tonwiedergabeanordnung (414), und zwar in Abhängigkeit von dem mathematischen Preis (c),

**dadurch gekennzeichnet, dass** die Aufbereitungseinheit (402) ein Audioverarbeitungsmittel (416) aufweist, vorgesehen zum Verarbeiten des Eingangs-Audiosignals (i) zum Herleiten das Ausgangs-Audiosignals (o) mit einer Wiedergabequalität (R) in Abhängigkeit von dem mathematischen Preis (c) und **dadurch gekennzeichnet, dass** die Wiedergabequalität wenigstens Folgendes umfasst:

(a) eine festgelegte Stereoqualität des Ausgangs-Audiosignals, d.h. ein Stereo-Audiosignal;
(b) eine Anzahl unabhängiger Ausgangssignale;
(c) eine Spezifikation einer Verteilung von Frequenzen des Ausgangs-Audiosignals; oder
(d) eine Amplitude des Ausgangs-Audiosignals.

**11.** Verfahren zum Herleiten eines Ausgangs-Audiosignals (o) aus einem Eingangs-Audiosignal (i), und zwar in Abhängigkeit von einem aus einer vom Benutzer beeinflussbaren Messung (m) hergeleiteten mathematischen Preis, **dadurch gekennzeichnet, dass** das Ausgangssignal (o) mit einer je nach dem mathematischen Preis (c) festgelegten Wiedergabequalität und **dadurch gekennzeichnet, dass** die Wiedergabequalität wenigstens Folgendes umfasst:

(a) eine festgelegte Stereoqualität des Ausgangs-Audiosignals, d.h. ein Stereo-Audiosignal;
(b) eine Anzahl unabhängiger Ausgangssignale;
(c) eine Spezifikation einer Verteilung von Frequenzen des Ausgangs-Audiosignals; oder
(d) eine Amplitude des Ausgangs-Audiosignals.

**12.** Computerprogramm zur Durchführung durch einen Prozessor, das das Verfahren nach Anspruch 11 beschreibt.

**13.** Datenträger (500), der das Computerprogramm nach Anspruch 12 speichert.

**Revendications**

**1.** Appareil de reproduction audio (200) comprenant :

- des moyens d'entrée (204) pour introduire un signal audio d'entrée (i) ;
- une sortie (206) pour émettre un signal audio de sortie (o) qui est dérivé du signal audio d'entrée (i) ;
- une entrée de coût (208) pour introduire un coût mathématique (c) qui est dérivé d'une mesure (m), laquelle mesure (m) est influençable par l'utilisateur ; et
- une unité de conditionnement (202) qui est capable de délivrer le signal audio de sortie (o) en fonction du coût mathématique (c),

**caractérisé en ce que** l'unité de conditionnement (202) comprend des moyens de traitement audio (216, 316) qui sont agencés de manière à traiter le signal audio d'entrée (i) pour dériver le signal audio de sortie (o) avec une qualité de reproduction (R) en fonction du coût mathématique (c) ; et **caractérisé en ce que** la qualité de reproduction (R) comprend au moins un des paramètres suivants :

(a) une qualité stéréo spécifiée du signal audio de sortie étant un signal audio stéréo ;
(b) un certain nombre de signaux de sortie indépendants ;
(c) une spécification d'une distribution de fréquences du signal audio de sortie ; et
(d) une amplitude du signal audio de sortie.

2. Appareil de reproduction audio (200) selon la revendication 1, dans lequel la qualité de reproduction (R) comprend une position tridimensionnelle d'une source sonore virtuelle (152), les moyens de traitement audio (216, 316) étant capables de simuler la source sonore virtuelle (152) au moyen du signal audio de sortie (o).

3. Appareil de reproduction audio (200) selon la revendication 2, dans lequel les moyens de traitement audio (316) comprennent un filtre (332) qui est agencé de manière à simuler la position de la source sonore virtuelle (152) en dérivant le signal audio de sortie (o) par le filtrage du signal audio d'entrée (i) avec une fonction de transfert liée à la tête dépendante de l'utilisateur (HRTF).

4. Appareil de reproduction audio (200) selon la revendication 2, dans lequel les moyens de traitement audio (316) comprennent une unité de traitement audio (342) qui est agencée de manière à simuler la position de la source sonore virtuelle (152) en changeant une propriété du signal audio de sortie (o) qui est sélectionné à partir d'une amplitude du signal et d'une réverbération ajoutée.

5. Appareil de reproduction audio (200) selon la revendication 1, comprenant une unité de calcul de première qualité (330) pour déterminer la qualité de reproduction (R) afin d'être utilisée dans la dérivation subséquente du signal audio de sortie (o) par les moyens de traitement audio (216).

6. Appareil de reproduction audio (200) selon la revendication 1, comprenant des moyens de mesure de la qualité (344) pour mesurer une mesure de qualité de sortie (M) du signal audio de sortie (o) et comprenant des moyens de calcul de la valeur paramétrique (346) pour calculer une valeur paramétrique (pv) pour être utilisée dans la dérivation subséquente du signal audio de sortie (o) par les moyens de traitement audio (216).

7. Appareil de reproduction audio (200) selon la revendication 1, dans lequel une unité de coût mathématique (310) est comprise qui est agencée de manière à dériver le coût mathématique (c) à partir de la mesure (m) qui est disponible d'un dispositif de mesure (312).

8. Appareil de reproduction (200) selon la revendication 7, dans lequel l'unité de calcul de coût mathématique (310) est agencée de manière à dériver le coût mathématique (c) sur la base d'une différence comprise entre la mesure (m) et une valeur choisie (d).

9. Appareil de reproduction audio (200) selon la revendication 7, dans lequel l'unité de calcul de coût mathématique (310) est agencée de manière à dériver le coût mathématique (c) à partir d'une mesure biométrique.

10. Système de rétroaction audio comprenant :

- une source audio (421) ;
- un dispositif de mesure (412) qui est agencé de manière à délivrer une mesure (m) qui est influençable par l'utilisateur ;
- une unité de calcul de coût mathématique (410) qui est agencée de manière à dériver un coût mathématique (c) à partir de la mesure (m) ;

- un dispositif de production sonore (414) ; et

- une unité de conditionnement (402) qui est agencée de manière à recevoir un signal audio d'entrée (i) en provenance de la source audio (421), à recevoir le coût mathématique (c) et à délivrer au dispositif de production sonore (414) un signal audio de sortie (o) qui est dérivé du signal audio d'entrée (i) en fonction du coût mathématique (c),

**caractérisé en ce que** l'unité de conditionnement (402) comprend des moyens de traitement audio (416) qui sont agencés de manière à traiter le signal audio d'entrée (i) afin de dériver le signal audio de sortie (o) avec une qualité de reproduction (R) en fonction du coût mathématique (c) ; et **caractérisé en ce que** la qualité de reproduction (R) comprend au moins un des paramètres suivants :

(a) une qualité stéréo spécifiée du signal audio de sortie étant un signal audio stéréo ;
(b) un certain nombre de signaux de sortie indépendants ;
(c) une spécification d'une distribution de fréquences du signal audio de sortie ; et
(d) une amplitude du signal audio de sortie.

11. Procédé de dérivation d'un signal audio de sortie (o) à partir d'un signal audio d'entrée (i) en fonction du coût mathématique (c) qui est dérivé d'une mesure (m) qui est influençable par l'utilisateur, **caractérisé en ce que** le signal audio de sortie (o) est dérivé avec une qualité de reproduction (R) en fonction du coût mathématique (c) ; et **caractérisé en ce que** la qualité de reproduction (R) comprend au moins un des paramètres suivants :

(a) une qualité stéréo spécifiée du signal audio de sortie étant un signal audio stéréo ;
(b) un certain nombre de signaux de sortie indépendants ;
(c) une spécification d'une distribution de fréquences du signal audio de sortie ; et
(d) une amplitude du signal audio de sortie.

12. Programme informatique pour être exécuté par un processeur qui décrit le procédé selon la revendication 11.

13. Support de données (500) qui mémorise le programme informatique selon la revendication 12.

FIG.1

FIG.2

FIG.3

FIG.4

FIG.5

FIG.6a

FIG.6b

P=α f(c)

700

90°

702

0°

right

left

c

# FIG.7

E

SPEC

FI1    FI2    FI3    FH    f

# FIG.8

**EP 1 582 090 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4788983 A
- JP 10207340 A
- US 6195434 B1
- US 4110918 A
- WO 0149066 A

### Non-patent literature cited in the description

- **F.L. Wightman ; D.J. Kistler.** Headphone simulation of free field listening. I: Stimulus synthesis. *Journal of the Acoustical Soc. of America,* February 1989, vol. 85 (2), 858-867